# EUROPEAN PATENT SPECIFICATION

(11) **EP 2 278 982 B1**
(45) Date of publication and mention of the grant of the patent: **26.10.2016**
(21) Application number: 09731179.9
(22) Date of filing: 09.04.2009
(51) Int. Cl.: A61K 35/56, A61P 25/28, A61K 33/06, A61K 33/00

(54) **COMPOSITIONS AND METHODS FOR TREATING NEURODEGENERATIVE DISEASES**
ZUSAMMENSETZUNGEN UND VERFAHREN ZUR BEHANDLUNG VON NEURODEGENERATIVEN ERKRANKUNGEN
COMPOSITIONS ET PROCÉDÉS DE TRAITEMENT DE MALADIES NEURODÉGÉNÉRATIVES

(30) Priority: 09.04.2008 US 43557 P
(43) Date of publication of application: 02.02.2011
(73) Proprietor: Marine Bio Co., Ltd., Tokyo 101-0036 (JP)
(72) Inventor: SOMEYA, Shinichi, Madison, WI 53705 (US)
(74) Representative: Glawe, Delfs, Moll
(86) International application number: PCT/US2009/040021
(87) International publication number: WO 2009/126778

(56) References cited:
- EP-A1- 0 790 004
- JP-A- 2004 113 150
- JP-A- 2004 262 849
- KR-B1- 100 658 950
- US-A- 4 540 584
- US-A1- 2003 104 007
- MARCASON WENDY: "What is the lowdown on Coral Calcium?", JOURNAL OF THE AMERICAN DIETETIC ASSOCIATION OCT 2003 LNKD- PUBMED:14520250, vol. 103, no. 10, October 2003 (2003-10), page 1319, XP002633458, ISSN: 0002-8223
- BLUMBERG S: "Is coral calcium a safe and effective supplement?", JOURNAL OF THE AMERICAN DIETETIC ASSOCIATION, THE ASSOCIATION, CHICAGO, IL, US, vol. 104, no. 9, 1 September 2004 (2004-09-01), pages 1335-1336, XP004549745, ISSN: 0002-8223, DOI: DOI:10.1016/J.JADA.2004.07.022
- GATES GEORGE A ET AL: "Presbycusis.", LANCET 2005 SEP 24-30 LNKD- PUBMED:16182900, vol. 366, no. 9491, 24 September 2005 (2005-09-24), pages 1111-1120, XP002633459, ISSN: 1474-547X
- FARID A. BADRIA ET AL.: 'Sarcophytolide: a new neuroprotective compound from the soft coral Sarcophyton glaucum.' TOXICOLOGY. vol. 131, no. 2-3, 16 November 1998, pages 133 - 143, XP001233802
- RAMOS A A ET AL: "Metal contents in Porites corals: Anthropogenic input of river run-off into a coral reef from an urbanized area, Okinawa", MARINE POLLUTION BULLETIN, OXFORD, GB, vol. 48, no. 3-4, 1 February 2004 (2004-02-01), pages 281-294, XP027424609, ISSN: 0025-326X, DOI: 10.1016/J.MARPOLBUL.2003.08.003 [retrieved on 2004-02-12]

## Description

### CROSS REFERENCE TO RELATED APPLICATIONS

This application claims priority to U.S. Provisional Patent Application Serial No.: 61/043,557, filed April 9, 2008.

### FIELD OF THE INVENTION

The present invention relates to compositions for use in methods for treating neurodegenerative diseases. The present invention provides compositions for treating and preventing presbycusis as defined in the claims.

### BACKGROUND OF THE INVENTION

Age-related hearing loss or presbycusis poses enormous humanitarian and economic challenges to society. In the US, it is estimated that 23 percent of people aged between 65 and 75 years of age, and 40 percent of people over 75 years of age have presbycusis, and the number of people suffering from this disorder is expected to grow dramatically as the population of older people increases (Someya et al., Neurobiol. Aging 20:1613 [2007]).

Presbycusis is the loss of hearing that gradually occurs in most individuals as they grow older. Hearing loss is a common disorder associated with aging. The loss associated with presbycusis is usually greater for high-pitched sounds or high frequency sounds.

There are many causes of presbycusis. Most commonly it arises from pathological changes in the inner ear of a person as he or she ages, but presbycusis can also result from pathological changes in the middle ear or from complex pathological changes along the nerve pathways leading to the brain. Presbycusis most often occurs in both ears, affecting them equally. Because the process of loss is gradual, people who have presbycusis may not realize that their hearing is diminishing. With presbycusis, sounds often seem less clear and lower in volume. This contributes to difficulty hearing and understanding speech.

Sensorineural hearing loss is caused by disorders of the inner ear or auditory nerve. Presbycusis is usually a sensorineural hearing disorder. It is most commonly caused by gradual changes in the inner ear. The cumulative effects of repeated exposure to daily traffic sounds or construction work, noisy offices, equipment that produces noise, and loud music can cause sensorineural hearing loss. Sensorineural hearing loss is most often due to a loss of hair cells (sensory receptors in the inner ear) and spiral ganglion cells (cochlear neurons). This can occur as a result of hereditary factors as well as aging, various health conditions, and side effects of some medicines (aspirin and certain antibiotics).

Presbycusis may be caused by changes in the blood supply to the ear because of heart disease, high blood pressure, vascular (pertaining to blood vessels) conditions caused by diabetes, or other circulatory problems. The loss may be mild, moderate, or severe.

Sometimes presbycusis is a conductive hearing disorder, meaning the loss of sound sensitivity is caused by abnormalities of the outer ear and/or middle ear. Such abnormalities may include reduced function of the tympanic membrane (the eardrum) or reduced function of the three tiny bones in the middle ear that carry sound waves from the tympanic membrane to the inner ear.

There are many strategies to help people with presbycusis. Hearing aids may be recommended for some individuals. Assistive listening devices can provide further improvement in hearing ability in certain situations. One example of such a device is the built-in telephone amplifier. Another example is FM systems that make sounds clearer, with or without a hearing aid, by delivering sound waves like a radio. Training in speechreading (using visual cues to determine what is being spoken) can help those with presbycusis to understand better what is being said in conversations or presentations. Nonetheless, no preventative or therapeutic interventions have been developed for presbycusis.

Coral water has been commercialized for use in the prevention or treatment of neurological diseases, see e.g. MARCASON WENDY: "What is the lowdown on Coral Calcium?", JOURNAL OF THE AMERICAN DIETETIC ASSOCIATION, vol. 103, no. 10, October 2003, page 1319 and BLUMBERG S: "Is coral calcium a safe and effective supplement?, JOURNAL OF THE AMERICAN DIETETIC ASSOCIATION, THE ASSOCIATION, vol. 104, no. 9, 1 September 2004, pages 1335-1336. Said cited prior art however is completely silent on the use of a composition comprising an extract of coral in the treatment or prevention of presbycusis as defined in the present invention as per the appended claims.

### SUMMARY OF THE INVENTION

The invention is set out in the appended claims. The embodiments of the description which do not fall within the scope of said claims are provided for illustrative purposes only and do not form part of the present invention.

The present invention relates to compositions for use in methods for treating neurodegenerative diseases. The present invention provides compositions for use in treating and preventing presbycusis as defined in the claims. The present description provides nutritional supplements, food products, and pharmaceutical compositions for use in methods for administering such products comprising compounds that prevent or reduce neurodegenerative diseases.

The present description provides compositions for use in methods for treating and/or preventing a neurodegenerative disease (e.g., presbycusis), comprising administering a composition comprising an extract of coral to a subject having symptoms of a neurodegenerative disease or at risk of a neurodegenerative disease under conditions such that symptoms are prevented or reduced. The coral extract may be an aqueous liquid (e.g., MiliQ water or distilled water). The coral extract may have a pH of approximately 7.0 to 9.0 (e.g., 8.0). The coral extract may have a calcium content of approximately 1.4 mg/L to 140 mg/L (e.g., 7 mg/L to 21 mg/L, 8.5 mg/L to 19.5 mg/L, 10 mg/L to 18 mg/L, 11 mg/L to 17 mg/L, 12.8 mg/L to 15.8 mg/L, 13.6 mg/L to 15 mg/L, 13.9 mg/L to 14.7 mg/L, 14 mg/L to 14.6 mg/L, 14.2 mg/L to 14.5 mg/L, or approximately 14.30 mg/L). The coral extract may have a magnesium content of approximately 0.54 mg/L to 54.0 mg/L (e.g., 2.7 mg/L to 8.1 mg/L, 3.2 mg/L to 7.6 mg/L , 3.8 mg/L to 7 mg/L, 4.3 mg/L to 6.5 mg/L, 4.9 mg/L to 5.9 mg/L, 5.1 mg/L to 5.7 mg/L, 5.2 mg/L to 5.6 mg/L, 5.3 mg/L to 5.5 mg/L, 5.35 mg/L to 5.45 mg/L, or approximately 5.4 mg/L). The coral extract may have a sodium content of approximately 0.1 mg/L to 50.0 mg/L (e.g., .53 mg/L to 1.59 mg/L, 0.76 mg/L to 1.46 mg/L, 0.86 mg/L to 1.26 mg/L, 0.96 mg/L to 1.16 mg/L, 1.0 mg/L to 1.1 mg/L, 1.03 mg/L to 1.09 mg/L, 1.04 mg/L to 1.08 mg/L, 1.05 mg/L to 1.07 mg/L, 1.055 mg/L to 1.065 mg/L, or approximately 1.06 mg/L). The coral extract may have a potassium content of approximately 0.03 mg/L to 30.0 mg/L (e.g., 0.14 mg/L to 0.42 mg/L, 0.17 mg/L to 0.53 mg/L , 0.2 mg/L to 0.36 mg/L, 0.22 mg/L to 0.34 mg/L, 0.25 mg/L to 0.31 mg/L, 0.27 mg/L to 0.29 mg/L, 0.275 mg/L to 0.285 mg/L, 0.272 mg/L to 0.288, 0.277 mg/L to 0.283 mg/L, or approximately 0.28 mg/L. The liquid may have a hardness of approximately 5.8 mg/L to 580 mg/L (e.g., 29 mg/L to 87 mg/L, 35 mg/L to 81 mg/L , 41 mg/L to 75 mg/L, 47 mg/L to 69 mg/L, 52 mg/L to 64 mg/L, 55 mg/L to 61 mg/L, 56 mg/L to 60 mg/L, 57 mg/L to 59 mg/L, 57.5 mg/L to 58.5 mg/L or approximately 58 mg/L. The administration of the coral extract may result in prevention or reduction in loss of spiral ganglion cells in the subject.

The coral liquid can be made by the method of immersing coral grains in purified water; and adjusting the pH of the water to 8.0±0.5. The coral liquid can be made by passing water over a water purification cartridge comprising coral grains (e.g., a home or industrial water purification system).

The present description additionally provides a composition for use in a method for treating a neurodegenerative disease, comprising administering an extract of natural stones with a pH of approximately 7.0 to 9.0 comprising a calcium content of approximately 1.4 mg/L to 140 mg/L, a magnesium content of approximately 0.54 mg/L to 54 mg/L, a sodium content of approximately 0.1 mg/L to 50.0 mg/L, a potassium content of approximately 0.03 mg/L to 30.0 mg/L, and a hardness of approximately 5.8 mg/L to 580 mg/L to a subject having symptoms of a neurodegenerative disease. The natural stones may be limestone, calcium carbonate, magnesium carbonate, sodium carbonate, potassium carbonate etc.

The present description additionally provides a composition for use in a method for treating a neurodegenerative disease, comprising administering an extract of chemical compounds with a pH of approximately 7.0 to 9.0 comprising a calcium content of approximately 1.4 mg/L to 140 mg/L, a magnesium content of approximately 0.54 mg/L to 54 mg/L, a sodium content of approximately 0.1 mg/L to 50.0 mg/L, a potassium content of approximately 0.03 mg/L to 30.0 mg/L, and a hardness of approximately 5.8 mg/L to 580 mg/L to a subject having symptoms of a neurodegenerative disease. The chemical compounds can be calcium carbonate, magnesium carbonate, sodium carbonate, potassium carbonate or a mixture of these compounds.

The present description further provides a food product, nutritional supplement, or pharmaceutical composition comprising an extract of coral in sufficient dosage to prevent or reduce neurodegenerative disease.

The invention is set out in the appended claims. The embodiments of the description which do not fall within the scope of said claims are provided for illustrative purposes only and do not form part of the present invention.

### DESCRIPTION OF THE FIGURES

Figure 1 shows an exemplary study design used in experiments conducted during the course of development of embodiments of the present invention.
Figure 2 shows the body weight of 20-month-old control and CW mice.
Figure 3 shows prevention of presbycusis by coral calcium water.
Figure 4 shows photomicrographs of the Cochlear Basal Turn from Control (A-C) and CW (D-F). Arrows indicate spiral ganglion and hair cells. Scale bar = 50 µm (left panel). Scale bar = 100 µm (middle and right panel).
Figure 5 shows prevention of spiral ganglion neuronal cell loss by coral-treated water. Error bars represent S.E.M.
Figure 6 shows prevention of hair cell loss by coral-treated water. Error bars represent S.E.M.

### DEFINITIONS

To facilitate an understanding of the present invention, a number of terms and phrases are defined below:
As used herein, the term "subject" refers to any animal (*e.g.,* a mammal), including, but not limited to, humans, non-human primates, rodents, companion animals (e.g., dogs, cats, etc.), and the like, which is to be the recipient of a particular treatment. Typically, the terms "subject" and "patient" are used interchangeably herein in reference to a human subject.

As used herein, the term "non-human animals" refers to all non-human animals including, but are not limited to, vertebrates such as rodents, non-human primates, ovines, bovines, ruminants, lagomorphs, porcines, caprines, equines, canines, felines, aves, etc.

As used herein, the term "*in vitro"* refers to an artificial environment and to processes or reactions that occur within an artificial environment. In vitro environments can consist of, but are not limited to, test tubes and cell culture. The term *"in vivo"* refers to the natural environment (e.g., an animal or a cell) and to processes or reactions that occur within a natural environment.

The terms "test compound" and "candidate compound" refer to any chemical entity, pharmaceutical, drug, and the like that is a candidate for use to treat or prevent a disease, illness, sickness, or disorder of bodily function (e.g., hearing loss). Test compounds comprise both known and potential therapeutic compounds. A test compound can be determined to be therapeutic by screening using the screening methods of the present invention.

As used herein, the term "sample" is used in its broadest sense. In one sense, it is meant to include a specimen or culture obtained from any source, as well as biological and environmental samples. Biological samples may be obtained from animals (including humans) and encompass fluids, solids, tissues, and gases. Biological samples include blood products, such as plasma, serum and the like. Environmental samples include environmental material such as surface matter, soil, water, and industrial samples. Such examples are not however to be construed as limiting the sample types applicable to the present invention.

"Prepared food product" means any pre-packaged food approved for human consumption.

As used herein, the term "physiologically acceptable carrier" refers to any carrier or excipient commonly used with oily pharmaceuticals. Such carriers or excipients include, but are not limited to, oils, starch, sucrose and lactose.

As used herein, the term "oral delivery vehicle" refers to any means of delivering a pharmaceutical orally, including, but not limited to, capsules, pills, tablets and syrups.

As used herein, the term "food product" refers to any food or feed suitable for consumption by humans, non-ruminant animals, or ruminant animals. The "food product" may be a prepared and packaged food (e.g., mayonnaise, salad dressing, bread, or cheese food) or an animal feed (e.g., extruded and pelleted animal feed or coarse mixed feed).

As used herein, the term "nutritional supplement" refers to a food product formulated as a dietary or nutritional supplement to be used as part of a diet.

As used herein, the term "mimetic" refers to a small molecule compound that mimics the binding or interaction of a ligand with its target or mimics the physiological effect of the compound. For example, a mimetic of a compound described herein is a small molecule that has the same physiological effect as the compound (e.g., prevention or treatment of neurodegenerative disease).

As used herein, the term "coral water" refers to the product of an extraction of coral exposed to a liquid or solvent. The coral may be fossilized coral grain harvested from the ocean floor. The coral may be powdered or otherwise dried prior to extraction. The liquid may be aqueous (e.g., water such as purified or distilled water). The extract may comprise salts and mineral. The coral extract may have a pH of approximately 7.0 to 9.0 (e.g., 8.0). Although knowledge of the exact composition is not required, in some embodiments, the extract comprises a calcium content of approximately 1.4 mg/L to 140 mg/L (e.g., 7 mg/L to 21 mg/L, 8.5 mg/L to 19.5 mg/L, 10 mg/L to 18 mg/L, 11 mg/L to 17 mg/L, 12.8 mg/L to 15.8 mg/L, 13.6 mg/L to 15 mg/L, 13.9 mg/L to 14.7 mg/L, 14 mg/L to 14.6 mg/L, 14.2 mg/L to 14.5 mg/L, or approximately 14.30 mg/L), a magnesium content of approximately 0.54 mg/L to 54.0 mg/L (e.g., 2.7 mg/L to 8.1 mg/L, 3.2 mg/L to 7.6 mg/L , 3.8 mg/L to 7 mg/L, 4.3 mg/L to 6.5 mg/L, 4.9 mg/L to 5.9 mg/L, 5.1 mg/L to 5.7 mg/L, 5.2 mg/L to 5.6 mg/L, 5.3 mg/L to 5.5 mg/L, 5.35 mg/L to 5.45 mg/L, or approximately 5.4 mg/L), a sodium content of approximately 0.1 mg/L to 50.0 mg/L (e.g., .53 mg/L to 1.59 mg/L, 0.76 mg/L to 1.46 mg/L, 0.86 mg/L to 1.26 mg/L, 0.96 mg/L to 1.16 mg/L, 1.0 mg/L to 1.1 mg/L, 1.03 mg/L to 1.09 mg/L, 1.04 mg/L to 1.08 mg/L, 1.05 mg/L to 1.07 mg/L, 1.055 mg/L to 1.065 mg/L, or approximately 1.06 mg/L), a potassium content of approximately 0.03 mg/L to 30.0 mg/L (e.g., 0.14 mg/L to 0.42 mg/L, 0.17 mg/L to 0.53 mg/L , 0.2 mg/L to 0.36 mg/L, 0.22 mg/L to 0.34 mg/L, 0.25 mg/L to 0.31 mg/L, 0.27 mg/L to 0.29 mg/L, 0.275 mg/L to 0.285 mg/L, 0.272 mg/L to 0.288, 0.277 mg/L to 0.283 mg/L, or approximately 0.28 mg/L, and a hardness of approximately 5.8 mg/L to 580 mg/L (e.g., 29 mg/L to 87 mg/L, 35 mg/L to 81 mg/L , 41 mg/L to 75 mg/L, 47 mg/L to 69 mg/L, 52 mg/L to 64 mg/L, 55 mg/L to 61 mg/L, 56 mg/L to 60 mg/L, 57 mg/L to 59 mg/L, 57.5 mg/L to 58.5 mg/L or approximately 58 mg/L.

### DETAILED DESCRIPTION OF THE INVENTION

The invention is set out in the appended claims. The embodiments of the description which do not fall within the scope of said claims are provided for illustrative purposes only and do not form part of the present invention.

The present invention relates to compositions for use in methods for treating neurodegenerative diseases. The present description provides compositions, pharmaceutical compositions, food products, nutritional supplements and methods of administering the compositions for prevention and treatment of neurodegenerative diseases such as presbycusis.

For example, experiments were conducted in vivo in a reliable animal model of presbycusis that shows late onset of age-related hearing loss by 15 months of age (Keithley et al., Hear Res. 188:21 [2004]) and significant loss of SGCs and hair cells by 12 months of age (Keithley et al., [2004], supra).

During experiments conducted during the course of development of embodiments of the present invention, the effects of coral-treated drinking water on presbycusis prevention and treatment in the animal model was evaluated. Hearing function tests revealed that control animals displayed severe hearing loss at 8, 16, 32 kHz, whereas coral-treated (CW) mice displayed normal hearing or mild hearing loss at 8, 16, 32 kHz (**P* < 0.05, *n* = 7), showing significant preservation of hearing function by coral-treated water. Histological analysis revealed that the cochleae from control animals displayed severe loss of spiral ganglion neurons and hair cells whereas the cochleae from CW animals displayed mild or less loss of the spiral ganglion neurons and hair cells, showing prevention of cochlear cell loss by coral-treated water. Moreover, the mean spiral ganglion cell (SGC) densities of apical, middle, and basal turns from CW animals were significantly higher than those of control animals, showing prevention of spiral ganglion neuronal cell loss by-treated water (**P* < 0.05, *n* = 4-5). The mean outer hair cell survival rate of middle and basal turns from CW mice were significantly higher than those of Control mice, showing prevention of outer hair cell loss by coral-treated water (**P* < 0.05, *n* = 4-5). Taken together, the experiments demonstrated that consumption of coral-treated water leads to prevention and treatment of presbycusis and prevents or treats physiological damage associated with a variety of neurodegenerative diseases and conditions. Accordingly, in some examples, the present description provides compositions for use in methods of treating and/or preventing neurodegenerative diseases such as presbycusis using coral extracts (e.g., water extracts).

### I. Coral Extracts

As described above, in some embodiments, the present invention provides compositions comprising coral extracts. In some embodiments, the extracts are in aqueous solutions. For example, in some embodiments, coral treated water is generated using coral grains or power. One exemplary non limited method is as follows: Coral grains or powder are immersed in distilled or pure water for a period of several minutes to several years (e.g., 1 hour to 1 year, 1 hour to 1 month, 1 hour to 1 week, 2 hours to 2 days, 12 hours to 36 hours, or approximately 24 hours) and then pH of the water is adjusted to 8.0±0.5 using NaOH or HCl. Since Coral grain is rich in minerals including calcium, magnesium, potassium, iron, zinc, selenium, manganese, chromium, etc., the coral-treated water is rich in these minerals and alkalic.

Any number of formulations of coral treated extracts are suitable for use in the compositions and methods of the present description. In some examples, administration is oral. The compositions of the present description may be provided in aqueous solution, oily solution, as or in any of the other forms discussed herein. The compositions may also be provided by any of a number of other routes, including, but not limited to, intravenous, intramuscular, intra-arterial, intramedullary, intrathecal, intraventricular, transdermal, subcutaneous, intraperitoneal, intranasal, enteral, topical, sublingual or rectal means. Further details on techniques for formulation for and administration and administration may be found in the latest edition of Remington's Pharmaceutical Sciences (Maack Publishing Co., Easton, PA).

In some examples, compositions are provided as a nutritional supplement alone or in combination with other ingredients. In some examples, nutritional supplements are packaged in a container or bottle that can include instructions describing dosage and timing of administration of the supplements.

In certain embodiments, the coral extracts of the present invention are provided as a food product comprising the extract. For example, in some embodiments, coral water is provided as bottled water for human or animal consumption. In other embodiments, coral extracts are added to prepared beverage products including, but not limited to, citrus juices, vegetable juices and like fruit beverages and vegetable juices, cola drinks, ginger ales, soda water and like carbonated beverages, sport drinks and like soft drinks, coffee, black tea, maccha and like tea-based beverages, cocoa, lactic acid bacteria beverages and like milk beverages, siruko, zenzai (thick azuki bean soup), nut (e.g., almond or peanut) beverages, malt beverages, hop beverages, tea-like beverages prepared from plants other than tea (barley tea, herb tea, Chinese matrimoneyvine tea, tochu (Eucommia ulmoides) tea), soymilk and soymilk-containing beverages, meal replacement beverages, energy drinks, diet drinks, and other general beverages.

In some examples, beverages comprise additional ingredients, including, but not limited to, carbonated water, food starch- e.g., modified, high fructose corn syrup and/or sucrose and/or sugar, sodium benzoate, caffeine, glycerol ester of wood resin, flavors, colors, preservatives, additional anti-oxidants, emulsifying agents, buffers, colorants, flavors, vitamins, minerals, nucleotides and nucleosides, pharmaceutical agents, thickening agents and stabilizers, and so forth. In some embodiments, compositions are packaged human use with suitable antioxidants such as lecithin, tocopherols, ascorbate, ascorbyl palmitate or spice extracts such as rosemary extract.

In some examples, beverages comprise vitamins. Examples include, but are not limited to water-soluble or oil-soluble, natural or synthetic, including various substances used as vitamin preparations. Examples of natural water-soluble vitamins include vitamin B1, B2, B6, B12, niacin, pantothenic acid, biotin, folic acid, lipoic acid, inositol, vitamin C and the like. Examples of natural oil-soluble vitamins include vitamin A, vitamin D, vitamin E, vitamin K and the like. Examples of the vitamin preparations commonly used as synthetic vitamins include vitamin A preparations such as retinol, vitamin D preparations such as ergocalciferol and dihydrotachysterol; vitamin B1 preparations such as thiamine hydrochloride and thiamine nitrate; vitamin B1 derivative preparations such as octothiamine, dicethiamine hydrochloride, thiamine disulfide, bisbentiamine, fursultiamine and benfotiamine; vitamin B2 preparations such as flavin adenine dinucleotide, riboflavin butyrate, riboflavin and riboflavin sodium phosphate; vitamin B6 preparations such as pyridoxine hydrochloride, pyridoxine phosphate and pyridoxal phosphate; vitamin B 12 preparations such as cobamamide, cyanocobalamin, hydroxocobalamin acetate, mecobalamin; vitamin E preparations such as tocopherol calcium succinate and tocopherol acetate; vitamin K preparations such as phyionadione, and menatetrenone.

In some examples, a composition comprising ingredients of coral extract is manufactured without the use of coral by adding ingredients to liquid (e.g., the coral extract compositions described above).

In some examples, extracts having the above described components are made from natural stones or chemical compounds (e.g., limestone, calcium carbonate, magnesium carbonate, sodium carbonate, potassium carbonate, etc), alone or in combination with other minerals.

In some examples, coral extracts are provided via a water purifying system that includes coral or other natural stones. For example home water purification systems (e.g., water pitcher filters, faucet filters, or under sink filters) may comprise a coral cartridge or coral component of a purification cartridge or filter. In other examples, industrial water filtrations systems (e.g., for the commercial production or purified water or beverages comprising purified water).

### II. Therapeutic Methods

The present description provides a composition for use in methods for prevention and treatment of presbycusis and other neurodegenerative diseases and conditions. For example, coral extracts (e.g., coral treated water) is administered to a subject to prevent presbycusis. In other examples, coral extracts are administered to a subject diagnosed with presbycusis as a treatment. In some examples, coral extracts are administered on a daily (or more or less often) basis for an extended period of time (e.g., weeks, months, or years). In some examples, coral treated water is provided as water bottles that are ready to drink. In other examples, coral extract is provided as a concentrated extract to be mixed with other beverages or food products (e.g., those described above).

In other examples, coral treated water is used to treat and/or prevent other diseases or signs or symptoms associated with neurodegeneration in the cochlea. For example, coral treated water is used to treat and/or prevent Alzheimer's disease, Parkinson's disease, Huntington's disease, amyotrophic lateral sclerosis, Alexander disease, Alper's disease, Ataxia telangiectasia, Batten disease (also known as Spielmeyer-Vogt-Sjogren-Batten disease), Bovine spongiform encephalopathy (BSE), Canavan disease, Cockayne syndrome, Corticobasal degeneration, Creutzfeldt-Jakob disease, HIV-associated dementia, Kennedy's disease, Krabbe disease, Lewy body dementia, Machado-Joseph disease (Spinocerebellar ataxia type 3), Multiple sclerosis, Multiple System Atrophy, Narcolepsy, Neuroborreliosis, Pelizaeus-Merzbacher Disease, Pick's disease, Primary lateral sclerosis, Prion diseases, Refsum's disease, Sandhoff disease, Schilder's disease, Subacute combined degeneration of spinal cord secondary to Pernicious Anaemia, Schizophrenia, Spielmeyer-Vogt-Sjogren-Batten disease (also known as Batten disease), Spinocerebellar ataxia (multiple types with varying characteristics), Spinal muscular atrophy, Steele-Richardson-Olszewski disease, and Tabes dorsalis.

### EXPERIMENTAL

### Example 1

### A. Materials and Methods

### 1.1. Water preparation

A method of making coral-treated and control water was as follows: 100 g of coral grains are immersed in 2 L of distilled water for 24 hr and then pH of the water was adjusted be 8.0±0.5 using NaOH or HCl. Control water was prepared using distilled water. pH of control water was adjusted to be 3.0±0.5 using NaOH or HCl since the standard drinking water for laboratory mice is acidified water whose pH is approximately 3.0.

### 1.2. Water composition

Calcium and magnesium contents were measured using an inductively coupled plasma emission spectrometer (*n* = 3). Sodium and potassium contents were measure using an atomic absorption spectrometry (*n* = 3). Data were examined using the two-tailed standard t-test. All data were reported as mean ± S.E.M.

### 1.3. Animals and dietary manipulation

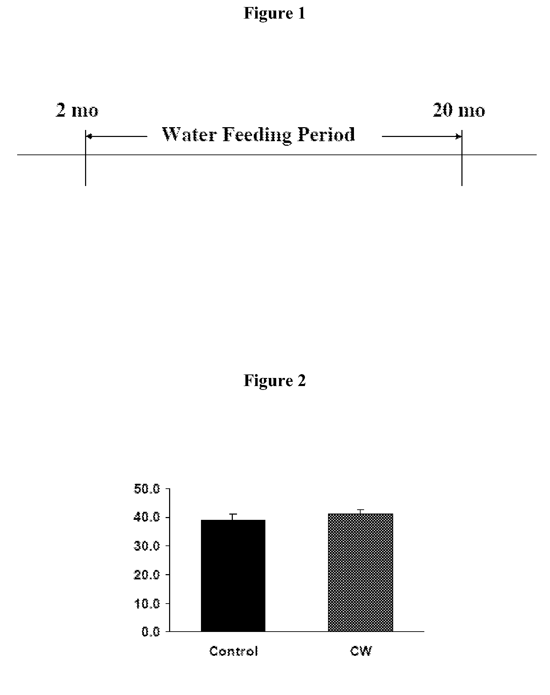

Details on the methods used to house and feed male inbred C57BL/6 (B6) mice have been described (Pugh et al., 1999). Briefly, mice were divided into two groups: control water (Control) and coral-treated water (CW). The CW treatment mice were fed the same diet and the same caloric intake as controls, but were fed with coral-treated water from 2 months of age until 20 months of age (Fig. 1). The control mice were fed with distilled and acidified water (pH3). Hearing tests were conducted at 20 months of age. The same animals were used for assessment of histology, spiral ganglion cell counting, and hair cell counting.

### 1.4. Assessment of hearing

Detailed protocols for ABR measurement have been described (Yamasoba et al., 2005). Briefly, ABRs were measured with a tone burst stimulus (4, 8, and 16 kHz) using an ABR recording system (Intelligent Hearing System). Mice were anesthetized with a mixture of xylazine hydrochloride (10 mg/kg, i.m.) and ketamine hydrochloride (40 mg/kg, i.m.). Seven mice per group were used for this study, and the same mice were used for the following histopathological analyses. Data were examined using the two-tailed standard t-test. All data were reported as mean ± S.E.M.

### 1.5. Histopathological analysis

Detailed protocols for tissue processing have been described (Someya et al., 2007). Briefly, 4% paraformaldehyde-fixed and paraffin-embedded specimens were sliced into 4 µm sections, mounted on silane-coated slides, stained with Haematoxylin and Eosin (HE), and observed under a light microscope (Leica Microsystems, Bannockburn, IL). The Rosenthal's canal was divided into three regions: apical, middle and basal (Keithley et al., 2004) and the three regions were used for evaluation of cochlear histopathology. Four to five mice for each group that were the same mice as those used in the ABR tests were used. Five modular sections obtained in every fifth section from one unilateral cochlea were evaluated per mouse. The same animals were used for spiral ganglion cell and hair cell counting.

Spiral ganglion cells (SGCs) were counted in each cross-section of the apical, middle, and basal turn of the cochlear individual sections using a 20X objective. Cells were identified by the presence of a nucleus. The corresponding area of Rosenthal's canal was measured on digital photomicrographs of each canal profile. The perimeter of the canal was traced with a cursor using ImageJ software (Rasband, W.S., ImageJ, U. S. National Institutes of Health, Bethesda, Maryland, USA, 1997-2007.). The computer then calculated the area within the outline. The SGC density was calculated as the number of SGCs per 1 mm². Five sections of the unilateral apical turn were evaluated in one cochlea per mouse. Data were analyzed using the two-tailed standard t-test. All data were reported as mean ± S.E.M.

Outer hair cell (OHC) and inner hair cell (IHC) were counted in each cross-section of the apical, middle, and basal turn of the cochlear individual sections using a 40X objective. Cells were identified by the presence of a nucleus. The outer hair cell survival rate (%) was calculated as follows. (# of OHCs/3) X 100. The inner hair cell survival rate (%) was calculated as follows. (# of OHCs/1) X 100. Five sections of the unilateral apical turn were evaluated in one cochlea per mouse. Data were analyzed using the two-tailed standard t-test. All data were reported as mean ± S.E.M.

### B. Results

### 2.1. Evaluation of coral-treated water

The calcium and potassium contents of CW were significantly higher than those of Control (Table 1) (**P* < 0.05, *n* = 5). The hardness and pH of CW were significantly higher than those of Control (**P* < 0.05, *n* = 3). Magnesium and Sodium were not detected in the Control water.

### 2.2. Evaluation of hearing function and cochlear histopathology

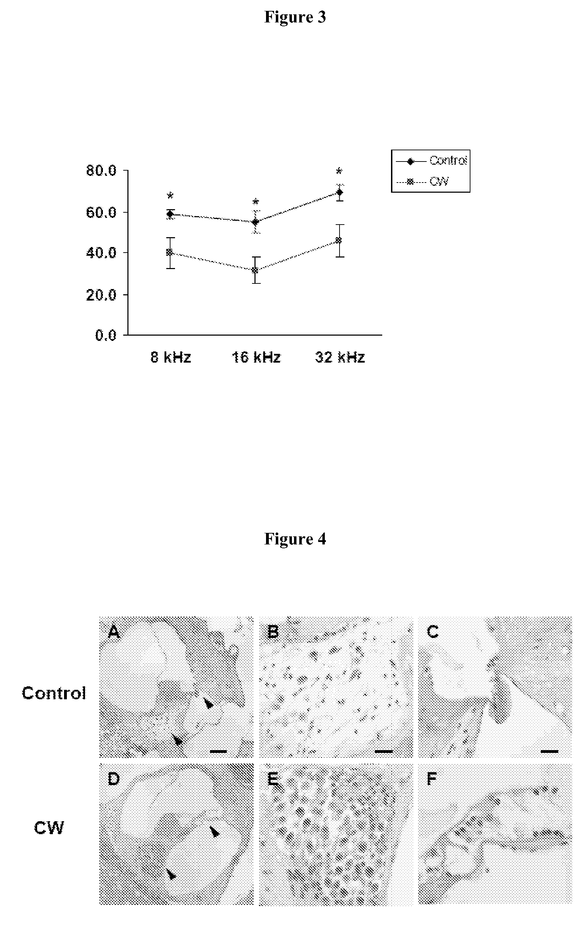

To examine the effects of long-term consumption of coral-treated water on presbycusis prevention and treatment, it was next examined whether the water retarded late onset of presbycusis in B6 mice. ABR thresholds measured from these mice revealed that Control mice displayed severe hearing loss at 8, 16, 32 kHz, whereas CW mice displayed normal hearing or mild hearing loss at 8, 16, 32 kHz (**P* < 0.05, n = 7), showing significant preservation of hearing function by coral-treated water (Fig. 3).

### 2.3. Evaluation of cochlear histopathology

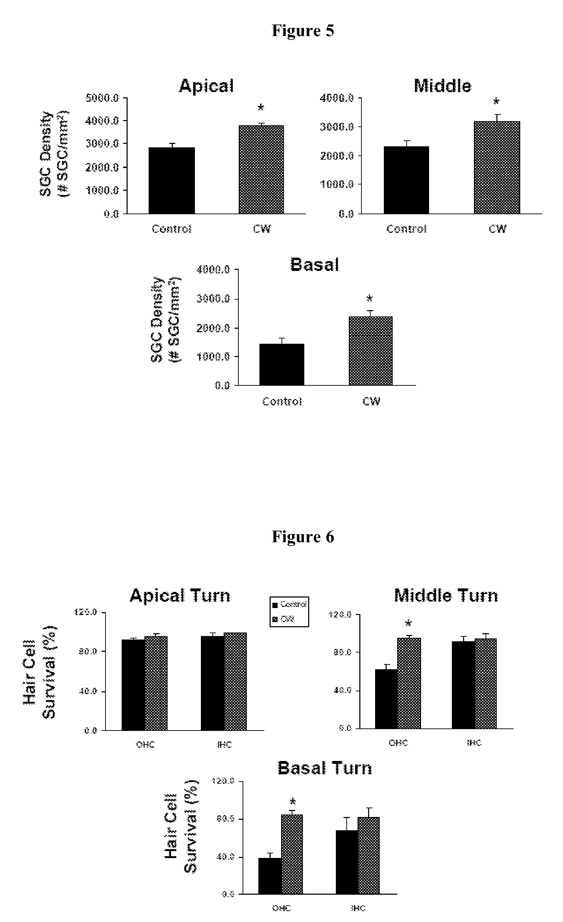

The effects of the water on age-related cochlear degeneration was next examined. The cochleae from Control mice (A) displayed severe loss of spiral ganglion neurons (B) and hair cells (C) whereas the cochleae from CW mice (D) displayed mild or only a few loss of the spiral ganglion neurons and hair cells, showing prevention of cochlear cell loss by coral-treated water (Fig. 4). The mean spiral ganglion cell (SGC) densities of apical, middle, and basal turns from CW mice were significantly higher than those of Control mice, showing prevention of spiral ganglion neuronal cell loss by-treated water (Fig. 5) (**P* < 0.05, *n* = 4-5). The mean outer hair cell survival of middle and basal turns from CW mice were significantly higher than those of Control mice, showing prevention of outer hair cell loss by coral-treated water (Fig. 6) (**P* < 0.05, *n* = 4-5).

**Table1. Water composition. The calcium and potassium contents of CW were significantly higher than those of Control. In addition, the hardness and pH of CW were significantly higher than those of Control Magnesium and Sodium were not detected in the Control.**

| | Control | CW |
|---|---|---|
| Ca (mg/L) | 2.47±0.77 | 14.30±1.78* |
| Mg (mg/L) | ND | 5.40±0.43 |
| Na (mg/L) | ND | 1.06±0.47 |
| K (mg/L) | 0.04±0.02 | 0.28±0.03* |
| Hardness (mg/L) | 6.33±1.86 | 58.00±6.26* |
| pH | 3.0±0.1 | 8.0±0.1* |

| | | |
|---|---|---|
| **P*<0.05, *n* = 3 | | |

## Claims

1. A composition comprising an extract of coral for use in a method for treating or preventing a neurodegenerative disease, wherein said neurodegenerative disease is presbycusis.

2. The composition for use according to claim 1, wherein said coral extract is an aqueous liquid.

3. The composition for use according to claim 1, wherein said coral extract has a pH of 7.0 to 9.0.

4. The composition for use according to claim 1, wherein said coral extract has one or more of the following features:
a calcium content of 1.4 mg/L to 140 mg/L,
a magnesium content of 0.54 mg/L to 54 mg/L,
a sodium content of 0.1 mg/L to 50.0 mg/L,
a potassium content of 0.03 mg/L to 30.0 mg/L.

5. The composition for use according to claim 2, wherein said liquid is selected from the group consisting of filtered water and distilled water.

6. The composition for use according to claim 5, wherein said liquid has a hardness of 5.8 mg/L to 580 mg/L.

7. The composition for use according to claim 5, wherein said coral liquid is made by the method of:
a) immersing coral grains in purified water;
b) adjusting the pH of the water to 8.0±0.5.

8. The composition for use according to claim 5, wherein said liquid is made by passing water over a purification cartridge comprising coral grains.

9. The composition for use of claim 1, wherein said coral extract is an aqueous liquid with a pH of 7.0 to 9.0 comprising a calcium content of 1.4 mg/L to 140 mg/L, a magnesium content of 0.54 mg/L to 54 mg/L, a sodium content of 0.1 mg/L to 50.0 mg/L, a potassium content of 0.03 mg/L to 30.0 mg/L, and a hardness of 5.8 mg/L to 580 mg/L.

10. A food product or pharmaceutical composition comprising an extract of coral in sufficient dosage for use in prevention or reduction of neurodegenerative disease, wherein said neurodegenerative disease is presbycusis, and wherein said extract of coral comprises a liquid with a pH of 7.0 to 9.0 comprising a calcium content of 1.4 mg/L to 140 mg/L, a magnesium content of 0.54 mg/L to 54 mg/L, a sodium content of 0.1 mg/L to 50.0 mg/L, a potassium content of 0.03 mg/L to 30.0 mg/L, and a hardness of 5.8 mg/L to 580 mg/L.

11. The composition for use of claim 10 or 9, wherein said liquid has a calcium content of 14.3 mg/L, a magnesium content of 5.4 mg/L, a sodium content of 1.06 mg/L, a potassium content of 0.28 mg/L, and a hardness of 58 mg/L.

## Patentansprüche

1. Zusammensetzung umfassend ein Korallenextrakt zur Verwendung in einem Verfahren zur Behandlung von oder Vorbeugung vor einer neurodegenerativen Krankheit, wobei die neurodegenerative Krankheit Presbyakusis ist.

2. Zusammensetzung zur Verwendung nach Anspruch 1, wobei besagter Korallenextrakt eine wässerige Flüssigkeit ist.

3. Zusammensetzung zur Verwendung nach Anspruch 1, wobei besagter Korallenextrakt einen pH-Wert von 7.0 bis 9.0 hat.

4. Zusammensetzung zur Verwendung nach Anspruch 1, wobei besagter Korallenextrakt eines oder mehrere der folgenden Merkmale hat:
ein Kalziumgehalt von 1,4 mg/l bis 140 mg/l,
ein Magnesiumgehalt von 0,54 mg/l bis 54 mg/l,
ein Natriumgehalt von 0,1 mg/l bis 50,0 mg/l,
ein Kaliumgehalt von 0,03 mg/l bis 30,0 mg/l.

5. Zusammensetzung zur Verwendung nach Anspruch 2, wobei besagte Flüssigkeit ausgewählt ist aus der Gruppe bestehend aus gefilterten und destillierten Wasser.

6. Zusammensetzung zur Verwendung nach Anspruch 5, wobei besagte Flüssigkeit eine Härte von 5,8 mg/l bis 580 mg/l hat.

7. Zusammensetzung zur Verwendung nach Anspruch 5, wobei besagte Korallenflüssigkeit hergestellt wird durch das Verfahren:
a) Eintauchen der Korallenkörnchen in gereinigtes Wasser;
b) Einstellung des pH-Werts des Wassers auf 8,0±0,5.

8. Zusammensetzung zur Verwendung nach Anspruch 5, wobei besagte Flüssigkeit hergestellt wird durch Leiten von Wasser über eine Korallenkörnchen umfassende Reinigungskartusche.

9. Zusammensetzung zur Verwendung nach Anspruch 1, wobei besagter Korallenextrakt eine wässerige Flüssigkeit mit einem pH-Wert von 7,0 bis 9,0 ist, ein Kalziumgehalt von 1,4 mg/l bis 140 mg/l, ein Magnesiumgehalt von 0,54 mg/l bis 54 mg/l, ein Natriumgehalt von 0,1 mg/l bis 50,0 mg/l, ein Kaliumgehalt von 0,03 mg/l bis 30,0 mg/l und eine Härte von 5,8 mg/l bis 580 mg/l hat.

10. Ein Lebensmittel oder eine pharmazeutische Zusammensetzung umfassend ein Korallenextrakt in einer ausreichenden Menge zur Verwendung bei der Vorsorge vor oder bei der Minderung einer neurodegenerativen Krankheit, wobei besagte neurodegenerative Krankheit Presbyakusis ist und wobei besagter Korallenextrakt eine Flüssigkeit mit einem pH-Wert von 7,0 bis 9,0 mit einem Kalziumgehalt von 1,4 mg/l bis 140 mg/l, einem Magnesiumgehalt von 0,54 mg/l bis 54 mg/l, einem Natriumgehalt von 0,1 mg/l bis 50,0 mg/l, einem Kaliumgehalt von 0,03 mg/l bis 30,0 mg/l und eine Härte von 5,8 mg/l bis 580 mg/l umfasst.

11. Zusammensetzung zur Verwendung nach Anspruch 10 oder 9, wobei besagte Flüssigkeit ein Kalziumgehalt von 14,3 mg/l, ein Magnesiumgehalt von 5,4 mg/l, ein Natriumgehalt von 1,06 mg/l, ein Kaliumgehalt von 0,28 mg/l und eine Härte von 58 mg/l hat.

## Revendications

1. Composition comprenant un extrait de corail pour une utilisation dans une méthode de traitement ou de prévention d'une maladie neurodégénérative, dans laquelle ladite maladie neurodégénérative est la presbyacousie.

2. Composition pour une utilisation selon la revendication 1, dans laquelle ledit extrait de corail est un liquide aqueux.

3. Composition pour une utilisation selon la revendication 1, dans laquelle ledit extrait de corail a un pH de 7,0 à 9,0.

4. Composition pour une utilisation selon la revendication 1, dans laquelle ledit extrait de corail a une ou plusieurs des caractéristiques suivantes :
une teneur en calcium de 1,4 mg/l à 140 mg/l,
une teneur en magnésium de 0,54 mg/l à 54 mg/l,
une teneur en sodium de 0,1 mg/l à 50,0 mg/l,
une teneur en potassium de 0,03 mg/l à 30,0 mg/l.

5. Composition pour une utilisation selon la revendication 2, dans laquelle ledit liquide est choisi dans le groupe constitué par de l'eau filtrée et de l'eau distillée.

6. Composition pour une utilisation selon la revendication 5, dans laquelle ledit liquide a une dureté de 5,8 mg/l à 580 mg/l.

7. Composition pour une utilisation selon la revendication 5, dans laquelle ledit liquide de corail est préparé par le procédé consistant à :
a) immerger des grains de corail dans de l'eau purifiée ;
b) ajuster le pH de l'eau à 8,0 ± 0,5.

8. Composition pour une utilisation selon la revendication 5, dans laquelle ledit liquide est préparé en faisant passer l'eau sur une cartouche de purification comprenant les grains de corail.

9. Composition pour une utilisation selon la revendication 1, dans laquelle ledit extrait de corail est un liquide aqueux avec un pH de 7,0 à 9,0 comprenant une teneur en calcium de 1,4 mg/l à 140 mg/l, une teneur en magnésium de 0,54 mg/l à 54 mg/l, une teneur en sodium de 0,1 mg/l à 50,0 mg/l, une teneur en potassium de 0,03 mg/l à 30,0 mg/l, et une dureté de 5,8 mg/l à 580 mg/l.

10. Produit alimentaire ou composition pharmaceutique comprenant un extrait de corail en une dose suffisante pour une utilisation dans la prévention ou la réduction d'une maladie neurodégénérative, dans lequel/laquelle ladite maladie neurodégénérative est la presbyacousie, et dans lequel/laquelle ledit extrait de corail comprend un liquide aqueux avec un pH de 7,0 à 9,0 comprenant une teneur en calcium de 1,4 mg/l à 140 mg/l, une teneur en magnésium de 0,54 mg/l à 54 mg/l, une teneur en sodium de 0,1 mg/l à 50,0 mg/l, une teneur en potassium de 0,03 mg/l à 30,0 mg/l, et une dureté de 5,8 mg/l à 580 mg/l.

11. Composition pour une utilisation selon la revendication 9 ou 10, dans laquelle ledit liquide a une teneur en calcium de 14,3 mg/l, une teneur en magnésium de 5,4 mg/l, une teneur en sodium de 1,06 mg/l, une teneur en potassium de 0,28 mg/l et une dureté de 58 mg/l.
